# EUROPEAN PATENT APPLICATION

(11) **EP 4 183 862 A1**
(43) Date of publication of application: **24.05.2023**
(21) Application number: 21208660.7
(22) Date of filing: 17.11.2021
(51) Int. Cl.: C12M 1/00, C12M 1/12

(54) **BIOMATERIAL FOR PRODUCING HYDROGEN**

(71) Applicant: AGFA NV, 2640 Mortsel (BE)
(72) Inventor: Strijckers, Hans, 2640 Mortsel (BE)
(74) Representative: Strijckers, Hans Louis P.

(57) **Abstract**

A biomaterial (1) for producing hydrogen including a first biolayer (4) deposited on a transparent plastic support (2) and a second biolayer (5) deposited on the first biolayer (4), wherein the first biolayer (4) contains a binder and photosynthetic microorganisms (6) and the second biolayer (5) contains a binder and aerobic fermentative bacteria (7).

## Description

### Technical Field

The present invention relates to biological materials and devices for producing hydrogen by microorganisms.

### Background Art

The challenges and opportunities of renewable energy have never been higher: cutting carbon levels (CO₂) in the atmosphere, improving our air quality, making full use of wind and solar resources and switching our transport systems to running on electricity and hydrogen.

Green algae are a very effective source of renewable energy production as they can grow at high rates. Algae have a harvesting cycle of 1 to 10 days and have been used for biofuel production from algal lipid. However, burning biofuel does not solve the problem of cutting carbon levels in the atmosphere.

Another intensely researched field of producing renewable energy is biological hydrogen production.

One way to produce biohydrogen is by fermentation of sugars and can be performed under aerobic or anaerobic conditions. For example, an anaerobic fermentative hydrogen production process is disclosed by US 2017226536 A (INDIAN OIL CORP LTD) .

Another way to produce biohydrogen is by photosynthesis. Also algae produce hydrogen under certain conditions. In 2000, it was discovered that if Chlamydomonas reinhardtii algae are deprived of sulphur they will switch from the production of oxygen, as in normal photosynthesis, to the production of hydrogen (see MELIS, Anastasios, et al. "Sustained Photobiological Hydrogen Gas Production upon Reversible Inactivation of Oxygen Evolution in the Green Alga Chlamydomonas reinhardtii. Plant Physiology. 2000 Jan, vol.122, no.1, p.127-136. and US 20010053543 A (US DEPARTMENT OF ENERGY)).

Photosynthesis in cyanobacteria and green algae splits water into hydrogen ions and electrons. The electrons are transported over ferredoxins. The enzyme Fe-Fe-hydrogenase combines them into hydrogen gas. The Fe-Fe-hydrogenases need an anaerobic environment as they are inactivated by oxygen. For the latter reason, a photobioreactor is used.

Intensive research is being performed to increase the efficiency of hydrogen production by photosynthetic microorganisms.

One approach involves genetic engineering of the algae. For instance, an improved solar energy conversion efficiency and photosynthetic productivity was found by using a truncated light-harvesting antenna size3 (tla3) DNA insertional transformant of Chlamydomonas reinhardtii (see HENNING, Kirst, et al. Truncated Photosystem Chlorophyll Antenna Size in the Green Microalga Chlamydomonas reinhardtii upon Deletion of the TLA3-CpSRP43 Gene. Plant Physiology. 2012 Dec, vol.160, no.4, p.2251-2260. ). Another example of genetic engineering to improve hydrogen production is given by CN 102776221 A (SHANGHAI INSTITUTE OF NUTRITION AND HEALTH) .

Another approach to increase hydrogen production is to seek synergies in co-cultures of algae and bacteria. An example of such research is disclosed in FAKHIMI, Neda, et al. Improving hydrogen production using co-cultivation of bacteria with Chlamydomonas reinhardtii microalga. Materials Science for Energy Technologies. 2019, vol.2, p.1-7.

Other research focusses on the design and the geometry of the photobioreactor (PBR), where the most relevant physical factors affecting the performance of the PBR are: light penetration, high area to volume ratio, temperature control, transpararency and durability of the material of construction gas exchange and agitation system. An overview of existing photobioreactors, such as tubular, flat plate and fermentor type PBR, is given in paragraph 6.3 of DASGUPTA, Chitralekha Nag, et al. Recent trends on the development of photobiological processes and photobioreactors for the improvement of hydrogen production. International Journal of Hydrogen Energy. 2010, vol.35, p.10218-10238.

US 2005176131 A (UNIVERSITIES OF IOWA AND MINNESOTA) discloses a composite biological device comprising a layered biostructure comprising at least one biological material embedded in a polymer layer and at least one additional porous layer that does not contain a biological material, wherein the biological material can produce H₂ gas and is not Thermotoga.

Nevertheless, there remains a need for further improvement in biohydrogen production especially on an industrial scale in an economically viable manner.

### Summary of invention

In order to overcome the problems described above, preferred embodiments of the present invention have been realized with a biomaterial (1) for producing hydrogen including a first biolayer (4) deposited on a transparent plastic support (2) and a second biolayer (5) deposited on the first biolayer (4), wherein the first biolayer (4) contains a binder and photosynthetic microorganisms (6) and the second biolayer (5) contains a binder and aerobic fermentative microorganisms (7).

The gist of the invention is that the aerobic fermentative microorganisms (7) immobilized in the second biolayer (5) consume oxygen for the fermentation of sugars and thereby create in the first biolayer (4) an environment that is poor on oxygen. In such hypoxic or anaerobic conditions the photosynthetic microorganisms (6) immobilized in the first biolayer (4) switch from the production of oxygen, as in normal photosynthesis, to the production of hydrogen.

A further benefit of the construction method of the biomaterial (1) is that a synergy can be created between the microorganisms of the first and second biolayer, resulting in substantially higher hydrogen production especially when the microorganisms in the first and second biolayer are algae respectively bacteria. For instance, Escherichia coli are bacteria that excrete acetic acid during the production of hydrogen from sugar fermentation. Accumulation of acetic acid causes the fermentation mechanism to stop and thus also the hydrogen production by E. coli. Green algae, like Chlamydomonas reinhardtii, can consume the excreted acetic acid. The result is a hydrogen production that is larger than when E. coli and C. reinhardtii would operate separately.

Another objective of the invention is the use of a photobioreactor containing the above biomaterial (1) for producing hydrogen.

These and other objectives will become apparent from the detailed description hereinafter.

### Brief description of drawings

**Figure 1** is a schematic representation of a biomaterial (1) in accordance with the invention showing a transparent support (2) having an adhesive layer (3) whereon a first biolayer (4) containing photosynthetic microorganisms (6) is deposited and where a second biolayer (5) containing aerobic fermentative microrganisms (7) is deposited on the first biolayer (4).
**Figure 2** is a schematic representation showing the same biomaterial (1) of Figure 1, but having in addition a backcoat layer (8) including spacers.
**Figure 3** is a schematic representation of a biomaterial (1) having on both sides of a transparent support (2), an adhesive layer (3) whereon a first biolayer (4) containing photosynthetic microorganisms (6) is deposited and where a second biolayer (5) containing aerobic fermentative microrganisms (7) is deposited on the first biolayers (4).
**Figure 4****.A** shows the absorption spectrum of chlorophyll a and b.

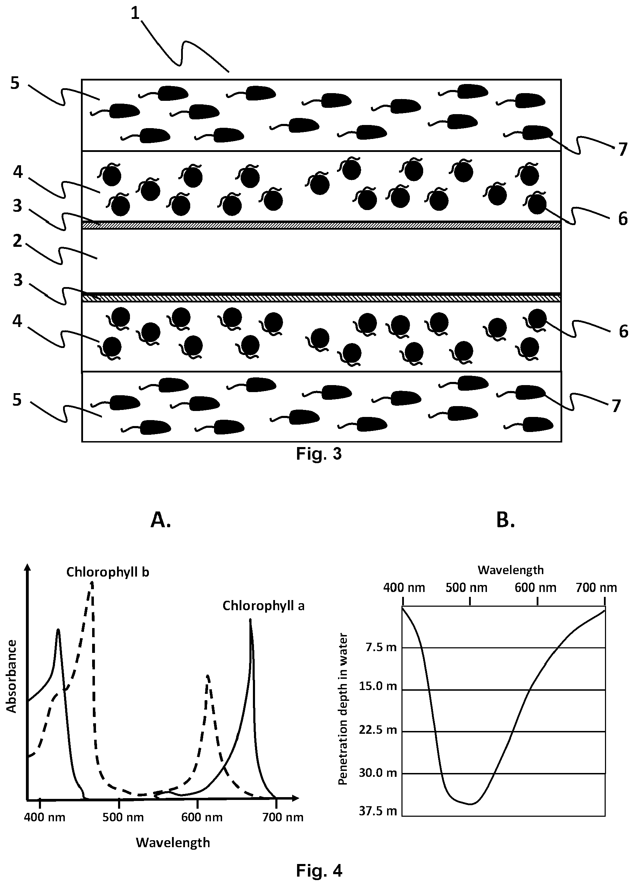

**Figure 4****.B** shows the penetration depth of light in water as a function of the wavelength of the light.

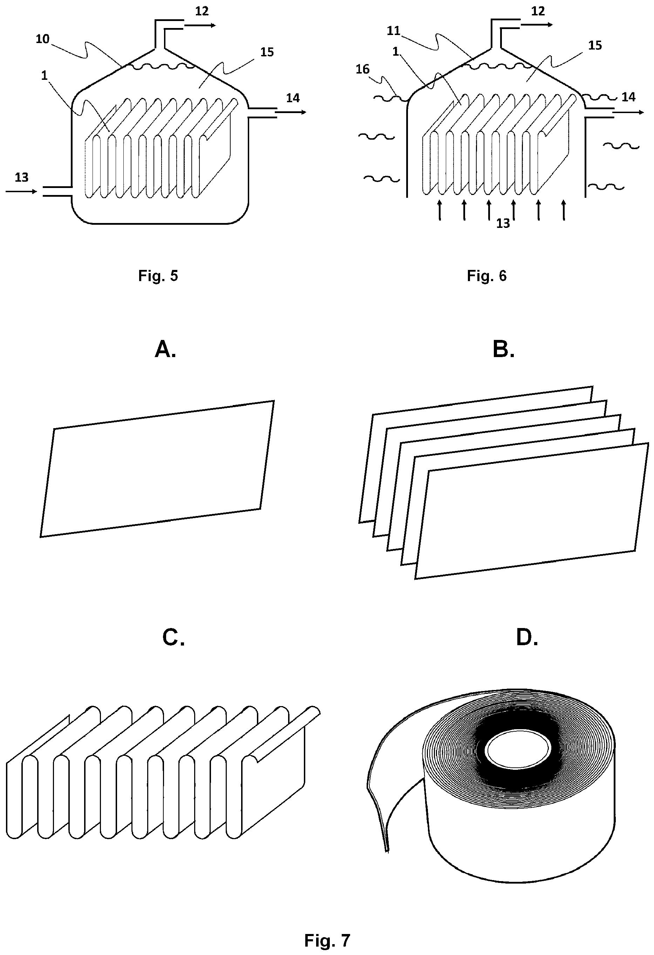

**Figure 5** shows a closed photobioreactor (10) containing a biomaterial (1) in an aqueous medium (15) having a hydrogen outlet (12), a nutrient inlet (13) and a waste outlet (14).
**Figure 6** shows a semi-closed photobioreactor (11) containing a biomaterial (1) in an aqueous medium (15) having a hydrogen outlet (12), a nutrient inlet (13) and a waste outlet (14). The semi-closed photobioreactor (11) is located in a pond having a water surface (16).
**Figure 7** shows examples of how the biomaterial (1) may be present in a photobioreactor, which include a flat sheet in Fig.7.A, a series of flat sheets in Fig.7.B, as a corrugated form in Fig.7.C or simply as a roll in Fig.7.D.

### Description of embodiments

### Biomaterials

A biomaterial (1) for producing hydrogen according to a preferred embodiment of the present invention includes a first biolayer (4) deposited on a transparent plastic support (2) and a second biolayer (5) deposited on the first biolayer (4), wherein the first biolayer (4) contains a binder and photosynthetic microorganisms (6) and the second biolayer (5) contains a binder and aerobic fermentative microorganisms (7).

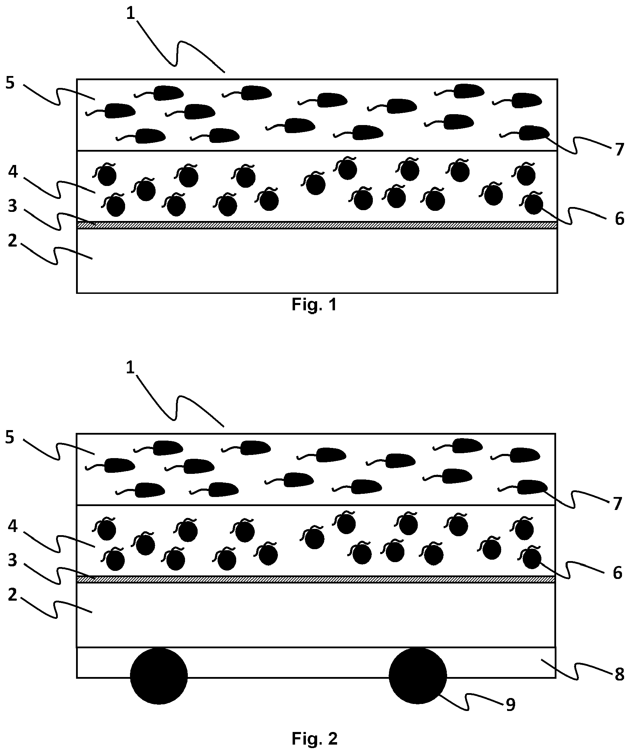

The biomaterial (1) preferably also includes one or more adhesive layers (3) for ensuring good adhesion of a first biolayer (4) to a transparent plastic support (2) as shown in Figure 1.

In one embodiment as illustrated by Figure 2, the biomaterial may also include a backcoat layer (8), for example for including an antistatic polymer and/or spacers (9). The spacers create a space between two layers, whereby a liquid flow can be organized between two biomaterials contacting each other. The antistatic polymer prevents the generation of electrical discharges, for example upon unwinding a roll coated with the first and second biolayers.

In another embodiment, as illustrated by Figure 3, the biomaterial (1) is a symmetrical material, meaning that on both sides of a transparent plastic support (2) it has a first biolayer (4) deposited on the transparent plastic support (2) and a second biolayer (5) deposited on the first biolayer (4), with preferably also one or more adhesive layers (3) between the transparent plastic support (2) and the first biolayer (4).

The first and second biolayers contain also a binder for providing a certain cohesive strength to the biolayers. Such cohesive strength is required to withstand aqueous liquids flowing against the surface of the biomaterial. To further increase this cohesive strength, the binder in the first and second biolayers is preferably crosslinked.

Another preferred protective measure is the application of a topcoat layer containing a binder on the second biolayer (5). The binder in the topcoat is preferably crosslinked.

### Supports

The biomaterial (1) includes a transparent support (2). The transparent support can be glass, but is preferably a transparent plastic support, more preferably a flexible transparent plastic support. Plastic supports have the advantage over glass that they are lightweight and not fragile. A flexible transparent plastic support has the further advantage that it can be handled more easily to take different shapes when constructing a photobioreactor.

The transparent plastic support preferably is made of a plastic that exhibits some durability when kept for a prolonged time in an aqueous medium or under sunlight exposure. For this reason of durability, the transparent plastic support is preferably made from a plastic selected from the group of polymethylmethacrylate (PMMA), polyethylene terephtalate (PET), polytetrafluorethene (PTFE or Teflon) and Polyvinylchloride (PVC). These transparent plastic supports are preferably in the shape of a plastic film having a thickness of less than 500 µm, more preferably for reasons of flexibility having a thickness of less than 200 µm.

Particularly preferred transparent plastic supports are made of PMMA or a polyester (such as PEN, PET and PLA), more preferably PET, and particularly preferably having a thickness between 80 and 200 µm for good flexibility.

The transparent plastic support preferably includes one or more adhesive layers on its surface. Suitable adhesive layers for plastic supports are well-known to the skilled person. For example, a preferred first adhesive layer (a), especially on a PET support, includes a vinylidene chloride-containing copolymer. The copolymer may be applied in the form of a latex and is preferably selected from vinylidene chloride-containing copolymers having carboxyl functional groups. Illustrative of such polymers are (1) copolymers of vinylidene chloride and an unsaturated carboxylic acid such as acrylic or methacrylic acid, (2) copolymers of vinylidene chloride and a half ester of an unsaturated carboxylic acid such as the monomethylester of itaconic acid, (3) terpolymers of vinylidene chloride, itaconic acid and an alkyl acrylate or methacrylate such as ethyl acrylate or methyl methacrylate, and (4) terpolymers of vinylidene chloride, acrylonitrile or methacrylonitrile and an unsaturated carboxylic acid such as acrylic acid or methacrylic acid.

In a preferred embodiment, the first adhesive layer is a layer including a latex polymer that is a co(vinylidene chloride-methyl acrylate-itaconic acid ; 88 % / 10 % / 2 %). This copolymer is prepared by emulsion polymerization using 0.5 % Mersolat^{™} H (BAYER) as emulsifying agent.

The dry thickness of this first adhesive layer is preferably about 0.1 µm.

A second adhesive layer (b) is coated on the above first adhesive layer (a). The second adhesive layer (b) preferably contains a hydrophilic polymer, more preferably a gelatin, most preferably a gelatin developed for photographic silver halide materials. The thickness of the gelatin-containing second adhesive layer (b) is preferably comprised between 0.1 and 1 µm.

### First biolayers

The first biolayer (4) includes a binder and photosynthetic microorganisms (6).

The photosynthetic microorganisms are preferably selected from green algae, blue-green algae(cyanobacteria) and photosynthetic bacteria.

Preferred photosynthetic microorganisms are green algae, more preferably Chlamydomonas ssp., Dunaliella ssp. and Chlorella ssp., and particular preferred are Chlamydomonas reinhardtii, Chlamydomonas moewusii, Dunaliella salina and Chlorella vulgaris.

Preferred photosynthetic bacteria include Rhodobacter sphaeroides, Rhodobacter capsulatus, Rhodobacter sulidophilus, Rhodopseudomonas sphaeroides, Rhodopseudomonas palustris, Rhodopseudomonas capsulate, Rhodospirillum rubrum, Chlorobium limicola, Chloroflexu aurantiacus, Thiocapsa roseopersicina and Halobacterium halobnium.

Preferred cyanobacteria are selected from Synechocystis ssp., Anabaena ssp. and Cyanothece ssp., more preferably Synechocystis PCC6803 and Anabaena variabilis.

Under aerobic conditions (presence of O₂), normal photosynthesis takes place wherein CO₂ is consumed with the aid of Chlorophyll:
6 CO₂ +12 H₂O + light energy → C₆H₁₂O₆ + 6 O₂ + 6 H₂O . This oxygen can be consumed by the aerobic fermentative microorganisms in the second biolayer, thereby depleting the first biolayer of oxygen and creating hypoxic or anaerobic conditions in the first biolayer.

Under anaerobic conditions (absence of O₂) the photosynthesis can, under certain conditions, follow a different pathway to produce hydrogen instead of oxygen. Such conditions are in place when Chlamydomonas reinhardtii is deprived from sulphur, as illustrated in MELIS, Anastasios, et al. "Sustained Photobiological Hydrogen Gas Production upon Reversible Inactivation of Oxygen Evolution in the Green Alga Chlamydomonas reinhardtii. Plant Physiology. 2000 Jan, vol.122, no.1, p.127-136. and US 20010053543 A (US DEPARTMENT OF ENERGY). For cyanobacteria, such as Plectonema boryanum, these conditions often require a nitrogen-limited environment.

Alternatively, one can also use copper addition rather than sulphur depletion to repress photosynthetic activity producing oxygen. An advantage of copper addition is that algae cells remained healthy, while with sulphur depletion it has been observed that algae cells stopped growing and died.

The amount of photosynthetic microorganisms present in the first biolayer depends on the light exposure. Preferably the photosynthetic microorganisms are present in the first biolayer in an amount of 0.1 to 10 g/m², more preferably 1 to 5 g/m².

The binder is preferably present in a weight ratio of the binder to the photosynthetic microorganisms of 3:1 to 1:3, more preferably 2:1 to 1:2.

### Second biolayers

The second biolayer (5) includes a binder and aerobic fermentative microorganisms (7). These microorganisms can produce hydrogen by fermentation of sugars and other substrates.

Fermentation is a process used by these microorganisms to generate energy where a suitable substrate is metabolized to make ATP by Substrate Level Phosphorylation (SLP). Fermentation pathways operate under anaerobic cell growth conditions when electron acceptors are unavailable to support cellular respiration (e.g., without O₂). Fermentation energy yields are low and as a result, cells grow more slowly than when they respire.

Important reactions start with glucose, which is converted to acetic acid:

C₆H₁₂O₆ + 2 H₂O -> 2 CH₃COOH + 2 CO₂ + 4 H₂.

Many fermenting bacteria including E. coli can convert acetyl-CoA to acetate to generate ATP. The two key enzymes are phosphate acetyltransferase and acetate kinase.

Aerobic respiration is a cellular process for harvesting energy. Electrons are extracted from an electron donor and transferred to O₂ as the terminal electron acceptor. This process generates a membrane potential across the cytoplasmic membrane termed proton motive force (pmf). The pmf is then used to drive ATP synthesis via the membrane-bound ATP synthase (electron transport phosphorylation). By respiration, oxygen is consumed (O₂ → CO₂). Also the oxygen in the first biolayer is consumed, whereby anaerobic conditions are created in the first biolayer.

Preferred fermentative bacteria include Enterobacter aerogenes, Enterobacter cloacae, Clostridum butyricum, Clostridum pasteurianum, Desulfovibrio vulgaris, Magashaera elsedenii, Citrobacter intermedius, Citrobacter amalonaticus and Escherichia coli.

In a particularly preferred embodiment, the first biolayer contains one or more green algae, preferably Chlamydomonas reinhardtii, while the second biolayer contains one or more aerobic fermentative bacteria, preferably Escherichia coli.

While the photosynthetic microorganisms (6) in the first biolayer (4) require light to produce hydrogen, this is not the case for the aerobic fermentative bacteria (7) in the second biolayer (5). This means that hydrogen production by the second biolayer can continue throughout the night or in dark light conditions. This mechanism is usually referred to as dark fermentation.

Numerous waste products contain a wide range of carbohydrates and are available at very low cost, allowing at the same time to purify waste water. These waste feedstock streams can be of industrial, agricultural, municipal or forestry origin. For most of these waste feedstock materials, pretreatments are necessary to liberate e.g. glucose by saccharification of the macromolecules. It should however be remembered that the pretreatment processes can also generate inhibitors and therefore the pretreatment method is to be carefully selected.

Preferably the aerobic fermentative microorganisms are present in the second biolayer in an amount of 0.1 to 10 g/m², more preferably 1 to 5 g/m².

The binder is preferably present in a weight ratio of the binder to the aerobic fermentative microorganisms of 3:1 to 1:3, more preferably 2:1 to 1:2.

### Binders and Crosslinkers

A binder is a polymer that gives a certain cohesive strength to the biolayers containing the microorganisms. Further cohesive strength is obtained by including also a crosslinker for the binder.

The binder is preferably a hydrophilic polymer, preferably selected from the group consisting of alginic acid, agar-agar, polyvinylalcohol, polyvinylpyrrolidone, gelatin and copolymers or mixtures thereof.

Alginic acid or algin is an edible polysaccharide obtained from brown algae. It is hydrophilic and forms a viscous gum when hydrated. When combined with cations it forms salts known as alginates. It is readily commercially available in powdered forms, because alginates are known as flavouring agents and are approved as food ingredients. The use of Ca²⁺ ions may be considered as a microorganism-friendly crosslinker for alginic acid or sodium alginate.

Agar-agar, or simply agar, is a jelly-like substance consisting of polysaccharides obtained from the cell walls of red algae species. Agar is a mixture of two components, the linear polysaccharide agarose and a heterogeneous mixture of smaller molecules called agaropectin. Agar is readily commercially available, even in supermarkets. Crosslinking of agar can be accomplished by using a diisocyanate, such as an aromatic diisocyanate like 4,4 diphenyl diisocyanate and/or aliphatic diisocyanate like 1,6 hexamethylene diisocyanate, with aromatic diisocyanates being preferred for physical properties (reactivity, tensile strength and thermal resistance). By the way, these diisocyanates can also be used to crosslink alginates.

Polyvinylalcohol is a non-toxic synthetic polymer having high tensile strength and flexibility, as well as high oxygen barrier properties. The latter makes it ideal to create hypoxic or anaerobic conditions in the first biolayer. Suitable polyvinylalcohols include Poval^{™} types from KURARAY. An efficient crosslinker for polyvinylalcohol is an aqueous solution of boric acid. Other crosslinkers include glutaraldehyde and difunctional carboxylic acids, such as suberic acid, and trifunctional carboxylic acids, such as citric acid.

Polyvinylpyrrolidone is a water-soluble polymer made from the monomer N-vinylpyrrolidone. Aqueous poly(vinyl pyrrolidone) solutions can be crosslinked by e.g. potassium persulfate and glutaraldehyde. Biocompatible crosslinking of polyvinylpyrrolidone copolymers can be accomplished with the methods disclosed in US 4692328 (DYNATECH) and US 4772484 (DYNATECH) .

Particularly preferred as binder is gelatin, especially photographic gelatin. Gelatin is a protein and serves as binder in silver halide photographic films. Silver halides are chemically sensitized with inter alia sulphur compounds for achieving good light sensitivity. However, gelatins also contained small amounts of sulphur due to their manufacturing process starting from connective tissue of pigs, cattle or fish. This resulted in a film sensitivity depending on the batch quality of gelatin. For achieving consistent film speeds, gelatin was purified to so-called photographic gelatin by eliminating sulphur and other impurities. In the present invention, gelatins and especially photographic gelatins are helpful to create an environment deprived of sulphur for the microorganisms so that the hydrogen production is not inhibited. Photographic gelatins are, for instance, available from ROUSSELOT.

Gelatin can be crosslinked with a wide variety of crosslinkers including compounds like glutaraldehyde and formaldehyde. Also divinylsulfon compounds can be used as illustrated by EP 0031959 A (AGFA).

However, crosslinkers may sometimes have disadvantageous effects to the microorganisms because of toxicity. For example, formaldehyde has a widespread application as a disinfectant for sterilization in the bacterial world.

Several methods exist to minimize these disadvantageous effects of crosslinkers on microorganisms.

A first method is to minimize the contact period with the microorganisms. The first and second biolayers can be coated with well-known coating techniques including curtain coating, knife or blade coating, forward-roll coating, reverse roll coating, slide coating, slot extrusion coating and the like. Instead of adding the crosslinker to the vessel containing the coating solution with microorganisms and binder, it is possible to formulate the crosslinker as a separate by-dosing solution which is mixed at the coating head with the microorganisms coating solution just prior to the coating thereof.

Another method is to apply the crosslinker in a separate layer, preferably as a topcoat on top of the second biolayer. The topcoat contains the crosslinker and preferably the same binder as the first and/or second biolayer. The crosslinker can then diffuse from the topcoat into the first and second biolayers and crosslink the binder in these layers during drying of the coated layers.

The above coating methods may also be combined with a selection method of the crosslinker.

The selection method of the crosslinker is a screening method, wherein different possible crosslinkers for a certain (co)polymer as the binder in the first and second biolayers are tested on the specific microorganisms to be used. The easiest way of testing the crosslinker on toxicity is to use a Petridish containing agar and the specific microorganism. The crosslinker with no or minimal toxicity can then sometimes even be used in the coating solution containing the binder and the microorganism(s) for the first and/or second biolayers.

For economical reasons, the first and second biolayers and the optional topcoat are preferably coated simultaneously.

For reasons of layer compatibility or crosslinking, in a preferred embodiment the same binder is used in the first and second biolayers and preferably also in the optional topcoat.

### Other ingredients

As desired or necessary, other compounds may be included in the coating solutions for the first and second biolayers and preferably also in the optional topcoat. Such compounds can include surfactants, nutrients for the microorganisms, inorganic salts, organic solvents, metal compounds, metal complexants, thickeners, biocides, pH adjusters (alkali/acids) and the like.

Surfactants can be useful for good equal spreading of the biolayers, especially on the adhesive layer.

Nutrients may be included in the biolayers. It is not the intention to grow the number of microorganisms, as thicker layers could possible result in clogging problems and provide obtructions for the aqueous flow in the photobioreactor. However, nutrients may be necessary to keep a steady state of the number of microorganisms in the biolayer.

The nutrients may be of an organic nature. Examples of suitable sugars and other organic nutrients include but are not limited to glucose, sucrose, saccharose, fructose, dextrose, lactose, sorbitol, xylitol, pectin, mannitol, manitose, erythritol, galactose, cellobiose, mannose, arabinose, ribose, erythrose, xylose, cyclodextrin, meso-erythritol, pentaerythritol, indulin, dextrin, polydextrose, maltose, maltodextrin of any DE, corn syrups, starch, amylose, amylopectin, pectic acid, cellulose and cellulose derivatives such as such as sodium-CMC, and hydroxypropylcellulose, galactomannans, guar gum, locust bean gum, gum arabic and the like.

In addition or alternatively, nutrients may be included in the aqueous medium (15) present in the photobioreactor (10, 11). Also polluted water containing organic waste can be used and has the advantage that simultaneously hydrogen is produced and water is being purified. Nevertheless, as already mentioned above, pretreatments are usually necessary for liberating sugars and care should be taken not to generate inhibitors for the hydrogen production. The aqueous medium or pretreated polluted water in the photobioreactor should be transparent or translucent at least when the first biolayer is exposed to light.

In addition to organic nutrients, also inorganic nutrients such as nitrogen (N), phosphorus (P), and potassium (K) may be included in the biolayer(s), if absent in sufficient amounts in the aqueous flow of the photobioreactor containing the biomaterial (1). Also metal ions of iron, as well as several trace elements, may also be considered important nutrients as the lack of one can limit the productivity of hydrogen.

Biocides or antibiotics that are harmful against other microorganisms than those of the first and second biolayers may be included to prevent that the other microorganisms become dominant over the microorganisms of the first and second biolayers.

### Photobioreactors

Hydrogen is a gas. Therefore, the biomaterial must be contained in some kind of a container allowing the capture of hydrogen. Furthermore, this container should allow (sun)light to reach the first biolayer. For using sunlight to directly access the first biolayer, this means that the container should be made of a transparent or at least a translucent material, preferably also exhibiting excellent durability in prolonged use of the biomaterial. A very suitable material for the photobioreactor is glass, because it has very high durability, is easy to clean and prevents damaging UV radiation to reach the microorganisms.

By including light sources in the container, an opaque material could also be used for the container, but this remains a less preferred embodiment. In such a case, the light sources are then preferably foreseen by electricity through the use of solar panels or wind energy. An example to include light sources in a PBR is given by DE 102014225349 A (TECHNISCHE UNIVERSITÄT DRESDEN ). An example of a solar powered photobioreactor is disclosed by US 2012288921 (GUANGZHOU INSTITUTE OF ENERGY) .

The above containers are usually referred to as photobioreactors. Such a photobioreactor(PBR) may be a closed or semi-closed container, but because hydrogen rises in water, at least the top part of the PBR is closed and provided with an outlet to collect the hydrogen gas. When using direct sunlight on the first biolayer, then this top part of the PBR is also transparent or at least translucent. Examples of a closed and a semi-closed photobioreactor are shown in Figure 5 respectively Figure 6.

Certainly if the PBR is a closed system, there should be an inlet for an aqueous flow of nutrients and an outlet for an aqueous flow of waste products.

In a preferred embodiment, a photobioreactor contains an aqueous medium (15) and the biomaterial (1) as described above and further includes a hydrogen gas outlet, an aqueous inlet and an aqueous outlet, and wherein the photobioreactor is at least partially transparent or translucent for sunlight.

From Figure 4, it should be clear that an aqueous medium (15) easily allows light to reach the biomaterial (1), more particularly the first biolayer wherein the photosynthetic microorganisms contain chlorophyll a and b. For a good light conversion by the photosynthetic microorganisms to hydrogen, the depth of the aqueous medium (15) in a photobioreactor is preferably no more than 5 m, more preferably no more than 2 m. Therefore, in a preferred embodiment, the width the biomaterial is no more than 2 m.

The biomaterial (1) may be included in a photobioreactor (10,11) in different shapes. In addition to good light penetration in the aqueous medium (15), also a high area to volume ratio of the biomaterial (1) is of interest. In **Figure 7** several examples are shown of a shape wherein the biomaterial (1) may be present in a photobioreactor. The biomaterial (1) can be present as a single sheet as in Fig.7.A, but preferably multiple sheets are present as Fig.7.B so that hydrogen production can be higher. A sheet or web of biomaterial (1) can also be bent to maximize light capture by the photosynthetic microorganisms in the first biolayer. This bending can take the form of a corrugated sheet as shown by Fig.7.C to increase the hydrogen production. Alternatively, the biomaterial can also be present as a roll (see Fig.7.D). A polyester or PMMA support may function as a light guide. wherein light is reflected in the support at the surfaces of the support. However, such light reflection is imperfect and a loss of light occurs. This loss of light is beneficial for the photosynthetic microorganisms as it is used for production of hydrogen.

The selection of the shape for the biomaterial may also depend on the geographical area where the biomaterial is used. For instance, in areas where sunlight is present in abundance, a roll of biomaterial may be preferred over a sheet, since too much sun exposure can put the microorganisms under stress or even kill them.

Care should also be taken not to impede the aqueous flow of nutrients and waste along the surface of the biomaterial. Therefore, a distance between two sheets or between two windings of a roll of biomaterial should be observed that does not impede this aqueous flow. Such a distance allowing aqueous flow is preferably between 0.1 mm and 10 cm, more preferably between 1 mm and 5 cm

Another factor to have attention for is the orientation of the biomaterial versus the light source. In the corrugated shape of Fig.7.C, sunlight can also penetrate the aqueous medium sideways if oriented in east-west direction, while at noon the sun is at its highest point in the sky and can expose the biomaterial from above.

The aqueous medium (15), for example between two sheets or between two windings of a roll of biomaterial, may contain small particulate matter capable of scattering light in multiple directions, thereby improving light capture by the photosynthetic microorganisms.

The skilled person is well aware of which nutrients should be present in the aqueous medium (15) and how the formulation should be adapted to maximize hydrogen production as illustrated by e.g. US 20170226536 (INDIAN OIL CORP) . Especially care should be taken that the first biolayer remains deprived of sulphur.

The aqueous medium (15) in a PBR is preferably maintained at a temperature in which the microorganisms thrive. Preferably the aqueous medium in a PBR is kept in a temperature range between 10°C and 40°C, more preferably between 15°C and 35°C. One way to accomplish these temperatures under hot air temperatures is to partially submerge the photobioreactor in a large volume of water, e.g. a pond. The latter is illustrated in Fig.6 by the water surface (16) on the outside of the semi-closed photobioreactor (11).

A PBR can operate in "batch mode" which involves replacing the aqueous medium in the reactor from time to time. However, preferably the PBR is operated in a continuous mode. Continuous operation requires control of all elements to prevent inactivation of the microorganisms. These elements included the provision of water, nutrients, air, and carbon dioxide at the correct rates. This allows the reactor to operate for long periods.

### Methods of manufacturing a biomaterial

A method of manufacturing a biomaterial (1) as described above includes the steps of: a) applying a first biolayer (4) on a transparent plastic support (2) and b) applying a second biolayer (5) on the first biolayer (4), wherein the first biolayer (4) contains a binder and photosynthetic microorganisms (6) and the second biolayer (5) contains a binder and fermentative bacteria (7).

The first and second biolayers are preferably applied with a coating technique selected from the group consisting of curtain coating, knife or blade coating, forward-roll coating, reverse roll coating, slide coating, and slot extrusion coating. By using one of these coating techniques, the biomaterial can be manufactured in an economical manner.

Alternatively, the first and second biolayers may be applied by a printing technology, such as flexography or inkjet printing.

### Methods for producing hydrogen

A photobioreactor containing an aqueous medium and the biomaterial as described above is preferably used for producing hydrogen.

The PBR is preferably a closed reactor, or at least semi-closed at the upper part of the reactor, having a hydrogen gas outlet for collecting the hydrogen gas.

A method of producing hydrogen with the above described biomaterial (1) in a photobioreactor includes at least the steps of: providing nutrients to an aqueous medium (15) and light to the biomaterial (1); and collecting hydrogen from an outlet in the photobioreactor. Preferably it also includes a step of discharging waste products from the photobioreactor. These steps may be repeated consecutively, but preferably occur continuously and/or simultaneously.

Not only the desired hydrogen gas, but also other undesired gases like CO₂ may be collected via the hydrogen outlet (12). This CO₂ can be the result of respiration by the aerobic fermentative microorganisms. But the skilled person is well aware of how to separate gases. For instance, US 2009305388 A (DRESSLER ) discloses how carbon dioxide can be eliminated from waste gases.

After hydrogen production, the biomaterial can be recycled. For example, a biomaterial on a PET support can be shredded into chips having a main surface area of approximately 1 cm². These chips can then be physically mixed with a caustic solution, e.g. a 2 % NaOH solution, at 85°C in a closed container to minimize evaporation of water and the caustic solution. This results in the biolayers being removed from the PET support. The PET chips can then be recycled by a sink float separation.

### Reference signs list

**Table 1**

| | |
|---|---|
| 1 | Biomaterial |
| 2 | Support |
| 3 | Adhesive layer |
| 4 | First biolayer |
| 5 | Second biolayer |
| 6 | Photosynthetic microorganisms |
| 7 | Aerobic fermentative microorganisms |
| 8 | Backcoat layer |
| 9 | Spacer |
| 10 | Closed PBR |
| 11 | Semi-closed PBR |
| 12 | Hydrogen outlet |
| 13 | Nutrient inlet |
| 14 | Waste outlet |
| 15 | Aqueous medium |
| 16 | Water surface |

## Claims

1. A biomaterial (1) for producing hydrogen including a first biolayer (4) deposited on a transparent plastic support (2) and a second biolayer (5) deposited on the first biolayer (4), wherein the first biolayer (4) contains a binder and photosynthetic microorganisms (6) and the second biolayer (5) contains a binder and aerobic fermentative bacteria (7).

2. The biomaterial according to claim 1 including one or more adhesive layers between the transparent plastic support (2) and a first biolayer (4).

3. The biomaterial according to claim 1 or 2, wherein the transparent plastic support (2) is a plastic film selected from polymethylmethacrylate (PMMA), polyethylene terephtalate (PET), polytetrafluorethene (PTFE or Teflon) and Polyvinylchloride (PVC).

4. The biomaterial according to any one of claims 1 to 3, wherein the photosynthetic microorganisms are selected from the group consisting of green algae and photosynthetic bacteria; and/or the aerobic fermentative microorganisms are selected from the group consisting of Enterobacter aerogenes, Enterobacter cloacae, Clostridum butyricum, Clostridum pasteurianum, Desulfovibrio vulgaris, Magashaera elsedenii, Citrobacter intermedius, Citrobacter amalonaticus and Escherichia coli.

5. The biomaterial according to any one of claims 1 to 3, wherein the first biolayer contains one or more green algae ande the second biolayer contains one or more aerobic fermentative bacteria, preferably Escherichia coli.

6. The biomaterial according to claim 5, wherein the first biolayer contains Chlamydomonas reinhardtii and the second biolayer contains Escherichia coli.

7. The biomaterial according to any one of claims 1 to 6, wherein the binder in the first biolayer (4) and/or the second biolayer (5) is selected from the group consisting of alginic acid, agar-agar, polyvinylalcohol, polyvinylpyrrolidone, gelatin and copolymers thereof.

8. The biomaterial according to any one of claims 1 to 7, wherein the binder is crosslinked.

9. The biomaterial according to any one of claims 1 to 8, wherein the biomaterial (1) carries on both sides of the transparent plastic support (2) a first biolayer (4) and a second biolayer (5) deposited on the first biolayer (4).

10. A photobioreactor containing the biomaterial according to any one of claims 1 to 9 including a hydrogen gas outlet, an aqueous inlet and an aqueous outlet, wherein the photobioreactor is at least partially transparent or translucent for sunlight.

11. The photobioreactor according to claim 10, wherein at least part of the biomaterial (1) is present in a plate shape.

12. The photobioreactor according to claim 10, wherein the biomaterial (1) includes multiple curved shapes.

13. A method of manufacturing a biomaterial (1) according to any one of claims 1 to 9 including the steps of:
a) applying a first biolayer (4) on a transparent plastic support (2) and
b) applying a second biolayer (5) on the first biolayer (4), wherein the first biolayer (4) contains a binder and photosynthetic microorganisms (6) and the second biolayer (5) contains a binder and aerobic fermentative microorganisms (7).

14. The method according to claim 14, wherein the first and/or second biolayer is applied by a coating technique selected from the group consisting of curtain coating, knife or blade coating, forward-roll coating, reverse roll coating, slide coating, and slot extrusion coating.

15. Use of a photobioreactor containing an aqueous medium and the biomaterial according claim 10 for producing hydrogen.
